# EUROPEAN PATENT SPECIFICATION

(11) **EP 3 396 567 B1**
(45) Date of publication and mention of the grant of the patent: **12.08.2020**
(21) Application number: 18169336.7
(22) Date of filing: 25.04.2018
(51) Int. Cl.: G08B 21/02, G08B 13/196, G16H 40/63, G16H 40/67, G16H 10/60

(54) **A WARNING SYSTEM AND METHOD FOR ISSUING A MESSAGE RELATING TO AN ALARM**
WARNSYSTEM UND VERFAHREN ZUR AUSGABE EINER NACHRICHT IN ZUSAMMENHANG MIT EINEM ALARM
SYSTÈME D'AVERTISSEMENT ET PROCÉDÉ POUR ÉMETTRE UN MESSAGE SE RAPPORTANT À UNE ALARME

(30) Priority: 27.04.2017 BE 201705297
(43) Date of publication of application: 31.10.2018
(73) Proprietor: Televic Healthcare NV, 8870 Izegem (BE)
(72) Inventor: De Smedt, Johannes Stefan M, 9032 Wondelgem (BE); Claeys, Bart Andre Simon, 8780 Oostrozebeke (BE)
(74) Representative: IP HILLS NV

(56) References cited:
- US-A1- 2008 205 311
- US-A1- 2008 263 050
- US-A1- 2011 071 880
- US-A1- 2012 030 133
- US-A1- 2014 218 517

## Description

### Technical Field

The present invention relates to a warning system for issuing a message with regard to an alarm. The alarm may be triggered by sensors or measurement instruments which monitor specific parameters or variables and generate an alarm signal when certain threshold values are exceeded, for example a smoke detector in a fire detection system, a heat, motion or contact sensor in an anti-intruder system, etc. The alarm may also be generated manually, for example by a patient or care-dependent person in a hospital or care home.

### Background

Warning systems that send an alarm state message to one or more terminals, for example a computer, tablet computer, smartphone, monitor, or other device of a user or in proximity to a user who is to be warned, are known. Such warning systems generate an alert message relating to the alarm state and send this alert message to one or more terminals of one or more users who are to be made aware of the alarm state. Existing warning systems comprise an alarm distribution module for this purpose. More advanced existing warning systems include a module or subsystem for the purpose of managing context information relating to the alarm state. Such a context information management system will for example send video images from a surveillance camera along with the alert message when an intrusion is detected.

In the United States patent application US 2004/0212505 A1 entitled "System and Method for Automatically Generating an Alert Message with Supplemental Information" context information, referred to as "a supplemental alert message component", is issued along with an alert message, which is sent in response to an emergency situation. Examples of an emergency situation cited in paragraph [0003] of US 2004/0212505 A1 are a broken window detected by a security system, a fall detected by a fall detection system, and a panic signal generated by a panic button. US 2004/0212505 A1 raises the problem of the information in an alert message relating to an emergency situation being too limited and solves this problem by automatically generating context information and sending it along with the alert message (see paragraph [0002] of US 2004/0212505 A1). Examples of such context information are provided in paragraphs [0024], [0028]-[0034], and [0041]: sensor data taken from one or more sensors, audio and/or video recordings, historical information relating to the person in need, location-specific information, information relating to actions taken in response to the emergency situation.

The context information that is sent along with the alert message in the warning system of US 2004/0212505 A1 increases the size of this alert message and thus makes it more difficult to send over a network. There is a real risk that the alert message will not reach the terminal of one or more users who are to be made aware of the emergency situation. Stated otherwise, the context information increases the risk that persons who are able to provide assistance, such as medical personnel, emergency services such as the fire brigade, friends or family, etc. are not alerted to the emergency situation in time, resulting in help not coming or coming too late.

The United States patent application US 2015/0049189 A1 entitled "Simultaneous Event Capture and Alert Generation" describes another warning system which integrates a traditional security system, in which security alarm messages are sent, with a traditional video surveillance system. In the warning system of US 2015/0049189 A1 the alarm messages and simultaneously received context information, such as images or footage from a surveillance camera, are stored together in a server, known as the data management server, which can be reached via a network. The information stored in the data management server can be consulted from a user device. The data management server may also alert the user device.

Since the warning system of US 2015/0049189 A1 must first upload the received alarm message and the simultaneously received context information together to a server before the information can be consulted by a user and/or before an alert message is sent to a user, the risk of a delayed reaction in US 2015/0049189 A1 is even greater. In any case, uploading the alarm message and the simultaneously received context information takes up time, which may be precious in the event of an emergency situation. Moreover, the network connection to the data management server may encounter problems resulting in the alarm message and the context information not reaching the data management server and thus no information being available to a helper and no alert message being sent to a helper.

The United States patent application US 2006/0010199 A1 entitled "Method and System for Providing Information to Remote Clients" describes yet another warning system in which audio/video is streamed to a user after confirmation is received from the user that an alert message arising from a sensor (e.g. a temperature, motion or light sensor) has reached him or her. The confirmation of receipt of the alert message from the sensor thus always results in audio and/or video (from a video camera, for example) being streamed.

In US 2006/0010199 A1 the user cannot simply just choose whether he or she wants to receive audio/video, how he or she wants to receive the audio/video, on which device he or she wants to receive the audio/video, or whether he or she wants to receive alternative context information or wants to receive no context information at all, etc.

United States patent application US 2008/0263050 A1 entitled "Decision Support Response Systems and Methods" describes a computer-implemented method for managing data in a clinical decision support system. The method includes generating and sending to a client system an alert e-mail message (step 68 in Fig. 3), receiving from the client system a request for additional information (step 70 in Fig. 3), obtaining the requested additional information (step 74 in Fig. 3) and transmitting the requested additional information to the client system (step 76 in Fig. 3).

Although US 2008/0263050 A1 resolves the problem of delayed alerting by obtaining and transmitting additional information only after a request for additional information has been received, the method known from US 2008/0263050 A1 does not provide means to inform the user of the client system on what additional information is available. According to paragraph [0024] of US 2008/0263050 A1, the user of the client system can use keywords, previously notified through training, in his request for additional information. The user however does not know if the additional information identified through previously learned keywords will be available in relation to a certain alert, and does not know if other information, not yet identifiable through a previously learned keyword, is available in relation to a certain alert. In addition, the use of previously learned keywords is error prone as the user may forget a keyword, mis-spell a keyword, mix-up keywords, etc.

United States patent application US 2014/0218517 A1 entitled "Home Monitoring Method and Apparatus" describes a home intrusion monitoring or security system. In case of an abnormal circumstance, an alarm message is sent to a mobile terminal. The alarm message may comprise positional information on the location of the abnormal circumstance. In response to the alarm message, a remote controlling app is launched on the mobile terminal, displaying camera images of the location where the abnormal circumstance occurred.

Although the alarm message contains positional information, the system known from US 2014/0218517 A1 does not inform the user on the availability of supplemental information in relation to the alarm, and it does not offer the user the possibility to select which supplemental information he/she desires to receive. As is indicated in paragraphs [0171]-[0172] of US 2014/0218517 A1, the app upon execution automatically downloads and displays the camera images for the location.

United States patent application US 2012/0030133 A1 entitled "Systems and Methods for Arranging Delivery of a Package" describes a system that facilitates a meeting between a package delivery vehicle and a package recipient. When the recipient is not home at the time of delivery of the package, the recipient receives an alert message including information on the vehicle's further route and stops. In response to the alert message, the recipient can select a stop point along the route where he/she can meet the delivery vehicle to exchange the packet.

United States patent application US 2011/0071880 A1 entitled "Location-Based Emergency Response System and Method" describes a system for identification and dispatching of a responder to an emergency situation. The responder is identified based on availability of the responder, proximity of the responder to the location of the emergency, and possibly a user profile as depicted in Fig. 2 of US 2011/0071880 A1 specifying the specialism of the responder.

### Summary of the Invention

The invention is defined by the appended claims. According to a first aspect, the invention relates to a warning system suitable for issuing a message relating to an alarming situation, such as defined in claim 1. Said warning system comprises:
- an alarm distribution module configured to generate an alert message relating to the alarming situation and to send said alert message to one or more terminals; and
- a context information management system configured to manage context information related to the alarming situation.

The alarm distribution module is adapted to generate a sublist of requestable context items related to the alarming situation and to include the sublist of requestable context items in the alert message, the sublist comprising a reference, indicator or short description of the requestable context items but not comprising the requestable context items themselves. The alarm distribution module is further adapted to generate the sublist on the basis of one or more of the following:
- terminal specifications of the terminal to which the alert message is sent;
- user profile specifications of a user of the terminal to which the alert message is sent; and
- legal restrictions.

The context information management system is adapted to deliver a context item from among the context information related to the alarming situation only after receiving an explicit request from a terminal.

The warning system according to the invention thus comprises an alarm distribution module which generates an alert message for an alert message and sends this alert message to one or more terminals which are to be made aware of the alarm state as quickly as possible. The alert message contains no context information so that no difficulty or delay is encountered when sending the alert message over a network. The warning system according to the invention does however include a context information management system in which context information connected to the alarm state is collected and stored. This context information may be of any type, such as for example a value, a set of values, a chart, a still image, a moving image, etc. The context information may also arise from various context information sources, such as for example sensors, information databanks, cameras, manually or automatically operated actuators, etc. The context information may be static or dynamic. Dynamic context information comprises for example streamed data or regularly refreshed data. In the warning system according to the invention, the context information management system will however send a context item to a user only when an explicit request therefor is received from the user, typically from the terminal to which the alert message is sent and after this alert message has been received and the user has thus definitely been made aware of the alarm state. In this way the user gains the flexibility to decide him- or herself which context information may be of use in making a decision in relation to the alarm state, and the flexibility to decide him- or herself on which device he or she wants to receive the context information. Context information which a user does not want to receive will not be sent over the network unnecessarily. Context information which a user does want to receive is sent but does not interfere with the transmission of the alert message, thereby avoiding the risk of the transmission of the alert message being late or unsuccessful.

To inform the user of a terminal to which the alert message is sent of the available context information that may be requested in connection with the alarm, a sublist of the requestable context items is composed and sent as part of the alert message. The sublist thus does not contain the requestable context items themselves but a reference, indicator or short description of the requestable context items. In this way, the size of the alert message is not substantially increased and thus the transmission and guaranteed delivery thereof to the terminal is not hindered or delayed. The terminal will, on reception of the alert message, extract and interpret the list of requestable context items, visually represent the requestable context items by, for example, pressable buttons on the touchscreen of the terminal for each of the requestable context items, and record which context items are actually requested by the user of the terminal. Thus, in the case of an alarm state in a patient room in a hospital for example, a sublist will be generated from which the blood pressure, the blood pressure history and camera images from the patient room may be requested.

The architecture with alarm distribution module and context information management system is modular and therefore has the additional advantage that new sources of context information may be added and existing sources of context information may be removed or modified without affecting the alarm distribution module. The basic functionality of the warning system, namely the transmission of alert messages at the moment of an alarm state, thus remains unchanged, while, irrespective thereof, sources of context information may be added, removed or modified.

The architecture with alarm distribution module and separate context information management system has the additional advantage that the context information management system may edit the context information before sending it to a terminal. The context information management system may thus act as a gateway and make the requested context information optimally suitable for viewing on the terminal to which the requested context information is to be sent. This comprises for example adapting the format, the resolution, the aspect ratio, etc.

In the warning system according to the invention, the alarm distribution module is adapted to generate the sublist on the basis of one or more of the following:
- terminal specifications of the terminal to which the alert message is sent;
- user profile specifications of a user of the terminal to which the alert message is sent; and
- legal restrictions.

Different alert messages which all relates to one and the same alarm state, but which are sent to different users and/or different terminals may thus contain a different sublist of requestable context items. The user profile specifies for example which access rights a user has to certain information. A carer will thus be able to request more limited or other information than a doctor in a warning system that monitors patients. The alarm distribution module may take this into account and thus insert, into the alert message sent to the carer, a sublist of requestable context items which differs from the sublist of requestable context items which is inserted into the alert message sent to the doctor. Thus, in the above example of a warning system for patients in rooms of a hospital or care facility, in the event of an alarm state a first alert message will be sent to the terminal of the carer, for example a monitor in a central room that is accessible to carers or the smartphone of the carer. A sublist is sent along with this first alert message, this sublist indicating that the blood pressure and/or a camera image from the patient room can be requested by the carer. At the same time, a second alert message is sent to the doctor, for example the cardiologist associated with the hospital or care facility. A sublist is sent along with this second alert message, this sublist indicating that the doctor may request the blood pressure and/or the blood pressure history of the patient. It may be important for the carer to inspect a camera image before proceeding to the room of the patient and potentially administering first aid there, whereas the blood pressure history contains information that is of little use to or even difficult to interpret for the carer. For the cardiologist, however, it is more important to be able to interpret the blood pressure history, whereas a camera image from the patient room is of little use here, since the cardiologist will not immediately proceed to the room, but will wait for confirmation of the alarm state by the carer.

The terminal specifications may also be observed when the sublist of requestable context items is composed. Thus, an alert message for an alarm state may be sent to the smartphone of a carer. This alert message may include a sublist in which only the blood pressure is given as a requestable context item because it was previously specified that no camera images are to be delivered to smartphones in general or because it was specified that no camera images are to be delivered to the specific smartphone of a specific carer. The reason may be that camera images require a certain type of application software or require certain bandwidths or volumes, the presence of which on smartphones should be assumed. Another alert message may be sent to a fixed monitor that can be consulted by the carer in a central room. The alert message may include a sublist in which both the blood pressure and a camera image from the patient room are listed as requestable context items, since the fixed monitor is assumed to have the required software and bandwidth to receive and to play the camera images.

Lastly, it is also possible for legal or judicial restrictions to play a role in determining which context items are included in the sublist for an alert message relating to an alarm state. Thus, it is for example possible that, for privacy reasons, camera images originating from a surveillance camera or from a camera in a patient room may not be delivered to certain persons, for example care staff, or may not be delivered to certain types of devices, for example monitoring or viewing screens which are visible on a care ward. The alarm distribution module will observe these restrictions when a sublist of requestable context items is generated for the purpose of being sent as part of an alert message relating to an alarm state.

One embodiment of the warning system according to the invention, defined in claim 2, further comprises:
- a receiver module in the terminal, wherein the receiver module is adapted to receive the alert message, to extract the sublist of context items related to the alarming situation from the alert message, and to provide the user of the terminal with the possibility of selecting one or more context items from the sublist.

Specifically, as mentioned above, the terminal will preferably be provided with a software application which is able to receive and interpret the alert message. In particular, this software application or receiver module will be able to extract the sublist of requestable context items from the alert message and be able to interpret it. The software application will create a screen in which the requestable context items are made available to the user so that the user may indicate which context items he or she wants to request, for example by pressing on one or more virtual buttons on a touchscreen. The user should thus provide each terminal, for example a smartphone, tablet computer, laptop, desktop, etc. on which he or she wants to receive alert messages relating to an alarm state with the receiver module, i.e. the software application able to receive and interpret such alert messages.

One embodiment of the warning system according to the invention, defined in claim 3, further comprises:
- a request module in the terminal, wherein the request module is adapted to generate a request message for one or more context items selected by the user, and to send the request message to the context information management system.

After the selection of context items by the user is known, a request module, which also forms part of the software application installed on the terminal of the user, will request the selected context items from the context information system, and the context information system will deliver the selected context items only after receipt of the explicit request therefor.

In one embodiment of the warning system according to the invention, defined in claim 4, said request module is adapted to also include, in the request message, one or more of the following:
- one or more parameters extracted from the alert message;
- terminal specifications of the terminal to which the selected context item is to be delivered;
- user preferences indicating an alternative terminal to which the selected context item is to be delivered.

Once a user has selected a context item, and this context item has been requested, the request module may automatically or semi-automatically also provide certain parameters. Thus, the terminal on which the user wants to receive the requested context item may be indicated. This may be the terminal on which the alert message relating to the alarm state was also received, but this does not necessarily have to be the case. The user may, after receiving an alert message relating to an alarm state on his or her smartphone, for example choose to receive a camera image on a large monitor present in the ward or room in which he or she is located. The ID of the monitor may be provided along with the request for the camera image so that the context information management system sends the requested camera image to the specified monitor instead of to the smartphone of the user. The request for a context item may be automatically or semi-automatically completed with the technical specifications of the terminal to which the context item will be delivered. The technical specifications comprise for example supported protocols, supported decoders, resolution of the screen available to this terminal, aspect ratio of the screen available to this terminal, etc. Additionally, the request for a context item may include one or more parameters which are extracted by the receiver module from the alert message. Typically, for example, an ID of the context item selected by the user will be retrieved from the sublist of requestable context items. This ID will then be sent along with the request message sent by the request module to the context information management system to explain which context item the user wants to receive.

In one embodiment of the warning system according to the invention, as defined in claim 5, different channels are used to send the alert message and to send the selected context item.

A selected context item will thus preferably be delivered via a channel that differs from the channel that is used to send the alert message. In this way interference between the transmission of context items and the transmission of alert messages is further precluded, thereby further strengthening the guarantee that the alert messages will be securely and promptly delivered.

In one embodiment of the warning system according to the invention, as defined in claim 6, the alarm distribution module will sequentially send said alert message:
- to the terminal via alternative channels;
- to alternative terminals associated with one and the same user;
- to alternative terminals in the vicinity of the user; and/or
- to alternative terminals associated with another user who is registered as a backup for said user,
until confirmation of receipt of the alert message by the user is received.

Specifically, one important advantage of the invention is that safeguards may be built in so as to guarantee that an alert message relating to an alarm state reaches a user. If no confirmation of receipt is received from the user, then the alert message may be sent again via an alternative channel to the terminal of the user, for example via SMS, via email, via a chat application, etc. if a confirmation of receipt is then still not received from the user, then the alert message may be sent again to one or more alternative terminals which are associated with the user, for example a smartphone, a tablet computer, a laptop computer, a desktop computer, a pager, etc. If a confirmation of receipt is then still not received, the alert message may be sent again to a terminal in the vicinity of the user, for example a monitor in the room in which the user is present, a home automation system which causes lighting in the room in which the user is present to flash, change colour, etc. The alert message may also be sent to the terminal or terminals of one or more users serving as a backup for the user who is to be reached, for example colleagues who work on the same care ward.

In one embodiment of the warning system according to the invention as defined in claim 7, said context information management system is adapted to link new sources of context information to the context information management system via plug-in.

Specifically, one important advantage of the invention is the flexible adaption thereof to new sources of context information. By virtue of the separation between alarm distribution module and context information management system, a new source of context information may be connected, via plug-in, to the context information management system, in which context information arising from this new source can also be requested without affecting the alarm distribution module which is responsible for guaranteeing delivery of alert messages relating to alarm states.

In one embodiment of the warning system according to the invention as defined in claim 8, the context information management system is adapted to receive context information once from a context information source, to save, to duplicate and to edit this context information to make variants of this context information which are delivered to one or more terminals in response to explicit requests therefrom.

Specifically, another important advantage of the invention is that the context information management system may act as a gateway which not only takes care of the collection and storage of context information related to alarm states, but which is also able to edit this context information so that it is delivered in optimal form to the terminal on which the context information is requested. Thus, the context information management system in the embodiments of the invention is able to adapt the resolution of images so that they may be optimally displayed on the screen of the terminal, to adapt the coding of context information to available decoders on the terminal, etc. The context information management system also acts as a buffer between the source devices delivering context information and a potentially large number of terminals which may simultaneously request the context information. It is common for a source device not to be able to cope with a large number of simultaneous requests. This is handled by the context information management system because it receives and processes the requests for context information, and is able to deliver the requested context information to the various terminals.

According to a second aspect, the present invention relates to a method, implemented on a computer, for warning about an alarming situation by issuing a message, as defined in claim 9, the method comprising:
- generating and dispatching an alert message relating to the alarming situation from an alarm distribution module to one or more terminals; and
- managing context information related to the alarming situation in a context information management system.

A sublist of requestable context items related to the alarming situation is generated and included in the alert message, the sublist comprising a reference, indicator or short description of the requestable context items but not comprising the requestable context items themselves. The sublist is further generated on the basis of one or more of the following:
- terminal specifications of the terminal to which the alert message is sent;
- user profile specifications of a user of the terminal to which the alert message is sent; and
- legal restrictions.

In the method according to the invention, a context item from among said context information related to said alarming situation will be sent by the context management system only after receiving an explicit request from a terminal from among the terminals.

According to a third aspect, the present invention relates to a computer program product as defined in claim 10, including instructions that can be run on a computer to carry out the method according to the invention when said program is run on a computer.

According to a fourth aspect, the present invention relates to a storage medium that can be read by a computer as defined in claim 11, said storage medium including the computer program product according to the invention.

### Brief Description of the Drawings

Fig. 1 illustrates one embodiment of the warning system according to the present invention; and
Fig. 2 shows a computer system suitable for embodying the central platform 101 in the warning system according to the present invention.

### Description of Embodiments

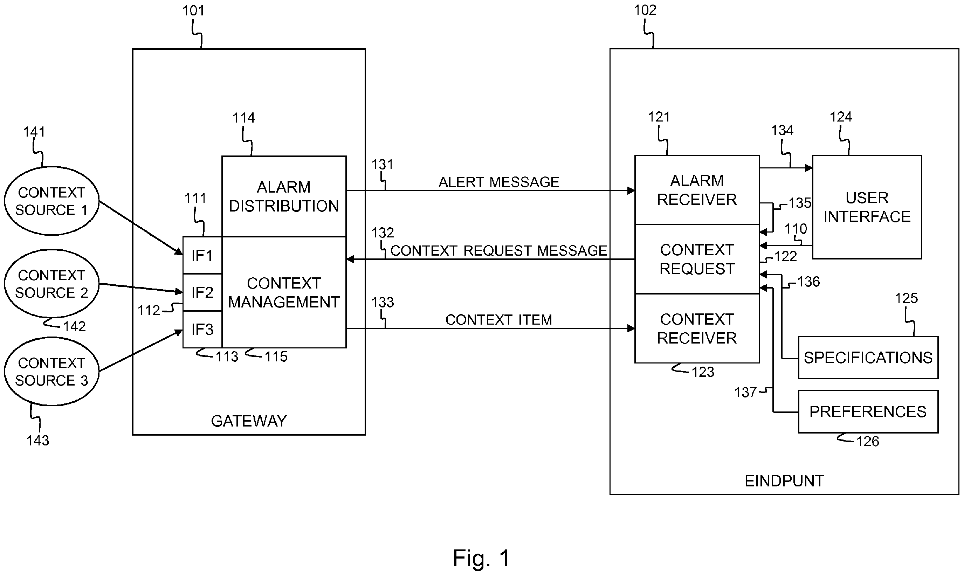

Fig. 1 shows an event embodiment of the warning system which is used in a hospital to warn care staff when an alarming situation or state occurs in a patient room. The warning system includes a central platform 101, also referred to as a gateway in Fig. 1. The warning system additionally comprises a software application or app which is installed on terminals of care staff. One of these terminals 102 is represented in Fig. 1. In alternative embodiments of the invention, a web application may be used. An advantage of such a web application is that no software application as to be installed on the terminal. The web application is logged in to from the terminal, whereupon the terminal functions. The central platform 101 includes an alarm distribution module 114, a context information management system 115 and a number of plug-in interfaces 111 or IF1, 112 or IF2, and 113 or IF3, which connect to respective context information sources 141, 142 and 143. The software application installed on the terminal 102 (or logged in to in the case of a web application) includes an alarm receiver module 121, a context request module 122, and a context receiver module 123. Fig. 1 further shows a user interface 124 of the terminal 102, for example a touchscreen if the terminal 102 is a smartphone with a touchscreen. Fig. 1 also further shows a memory 125 in which technical specifications of the terminal 102 are stored, such as for example the screen resolution of the touchscreen 124, the aspect ratio of the touchscreen 125, decoders supported by the terminal 102, etc. Lastly, Fig. 1 also shows a memory 126 in which user preferences of the user of the terminal 102 are stored, such as for example the preference to receive video images on an alternative terminal, for example the tablet computer of this same user.

In the following example it is assumed that the first context information source 141 is a medical thermometer, the second context information source is a database in which blood pressure measurements arising from a blood pressure sensor are stored, and the third context information source is a video camera present in the patient room. The body temperature of the patient in the patient room is tracked. As soon as the body temperature exceeds a specific threshold, for example 40°C, the alarm distribution module 114 becomes aware that there is an alarm state. The alarm distribution module immediately generates an alert message 131 which is sent to the terminals 102 of the care providers or other persons who are to be informed of the alarm state. These may be the carers working on the floor of the patient room, but also for example a doctor, a cardiologist, etc. Included in the alert message 131 is a sublist of context information relating to the alarm state which may be requested by the user to whom the alert message is sent. Available to the context information management system 115 in the concrete example provided above is for example the measured body temperature, since this was received from the medical thermometer 141. Also available to the context information management system are the blood pressure values of the patient. These are received from the sphygmomanometer 142, stored locally in the context information management system 115, and may be presented in the form of a table or graph to users capable of interpreting such information. Lastly, available to the context information management system 115 is video footage produced by the camera 143 in the patient room immediately following the alarm state. There are thus three different context items with relation to the alarm state. The context information management system 115 will not provide all three context items to each of the users to which an alert message is sent. Thus, care staff will not be presented with the possibility of requesting the blood pressure history, and the cardiologist will not be presented with the possibility of viewing the camera images from the patient room. The context information management system 115 will compose sublists of requestable context information for different users or different types of users. In a first alert message 131 sent to the terminal of a carer working on the floor of the patient room, a sublist will be included which consists of the measured body temperature and camera images from the patient room. In a second alert message sent to the terminal of the cardiologist, a sublist will be included which consists of the measured body temperature and the blood pressure history. The alert message 131 is received by the alarm receiver module 121 in the terminal 102. The alarm receiver module 121 extracts the sublist 134 of requestable context information and passes it on to the user interface 124 so that the user of the terminal 122 is presented with the possibility of requesting the respective context items from the sublist 134. The user interface 124 will for example show a button on the touchscreen for each context item from the sublist 134. When this button is touched, the user interface 134 will pass an instruction 110 on to the context request module 122. The context request module 122 generates a context request message 132 in which the delivery of a specified context item is explicitly requested. Depending on the configuration of the user interface 124, it is of course also possible for multiple context items from the sublist 134 to be requested via a single context request message 132. In the concrete example provided above, the carer whose terminal 102 has received the alert message 131 will indicate that he or she wants to receive camera images from the patient room. The context request module 122 thus generates a context request message 132 in which it is explicitly requested that the camera images relating to the alarm be sent to the carer. To identify the alarm state for which the camera footage is requested, certain parameters 135 from the received alert message 131 are included in the context request message 132. It is additionally possible for certain specifications 136 of the terminal to which the camera footage is to be sent to be included in the context request message 132. Thus, the memory 125 may contain information regarding the video codecs which are supported by the terminal 122 and the resolution of the screen 124 on which the camera images will be viewed. The context information management system 132 receives and interprets the context request message 132. Next, the context information management system will select and edit the camera images in order to send it to the terminal 102 in accordance with the provided specifications. The camera images are thus sent as a context item 133 in response to the received context request 132. The camera images are optionally edited beforehand so that they are delivered with the optimal resolution for playback on the screen 124. The camera images are also coded using a codec supported on the terminal 125 so that the camera images can be decoded there before they are played back. The context receiver module 123 receives the camera images 133 and passes them on to the decoder which decodes the camera images so that they may be played back on the terminal.

When the alert message 131 is sent over a first communication channel, for example as an SMS message over a 3G mobile network, then the requested context information - in the above example the camera images - will typically be delivered over another communication channel, for example the Wi-Fi network of the hospital in which the patient and carer are located. It is of great importance that the alert message is securely received on the terminal, as in the event of an alarm state, it is expected that certain users are at least made aware of the fact that there is an alarm state or emergency situation. In the absence of confirmation of receipt, attempts will sequentially be made to send the message to the terminal 102 via alternative channels. If these are also unsuccessful, attempts may be made to send the alert message to alternative terminals associated with the same user in the alarm distribution module 114. Thus, an attempt may be made to send the alert message to the tablet computer of the carer, or the desktop computer of the carer in the event that no confirmation of receipt is received from the smartphone 102 of the carer for the alert message 131. If a confirmation of receipt is also not received from the alternative terminals within a determined time period, then attempts may still be made to send the alert message 131 to alternative terminals that are likely to be located in the vicinity of the carer, such as for example a large screen in the operating theatre in which the carer is located, or lighting in the room in which the carer is located so that the carer is alerted, by flashing lights for example, to the fact that there is an emergency situation. The alert message may also be sent to another user who is registered as a backup in the system, for example a colleague working on the same care ward. Guaranteed delivery is less critical for the context information 133. For this purpose, channels will thus preferably be used which do not interfere with the channel used to send the alert message 131, and typically no confirmation of receipt will be requested. A context item will however only be sent after the user has specifically requested this in order to affect the transmission of the more critical alert message as little as possible.

A first remark in relation to the above embodiment of the invention is that, in an alternative scenario, the camera images 133 may also be delivered to an alternative terminal, for example a terminal other than the smartphone 102 from which the carer requested the camera images. This will for example be the case if the user preferences 126 specify that the user prefers to receive video images on another terminal, such as his or her tablet computer, so that the images may be viewed on a larger screen, and potentially with a higher resolution. The screen size, the desired resolution and potential other technical specifications of this alternative terminal on which the camera images 133 are wanted, may be specified in the context request message 132, or these technical specifications may be programmed beforehand and stored in the context information management system 115.

It should also be noted that, in advanced embodiments of the invention the central platform 101, more specifically the context information management system 115, also keeps user profiles for individual users or types of users. The user profiles stipulate which context information will be made available to which users in relation to certain alarm states. In the user profiles, it is possible to take into account the role or responsibility held by a certain type of user in an organisation (a carer is presented with other context information than a doctor or specialist), the personal preferences of individual users (such as for example the preferences which are also stored locally in the memory 126 of the terminal of a user), legal restrictions, such as privacy legislation, which may depend on the country or region, etc.

One important advantage of the warning system illustrated in Fig. 1 is the modularity and flexibility of adding or removing new context information sources. Plug-in interfaces for new sources such as IF1, IF2 and IF3 allow new sources, for example extra cameras, sensors, wearables, etc. to be connected, which generate useful information in relation with alarm situations. The information arising from such new context information sources may be imported via the plug-in interface and stored in the context information management system 115. The context information management system includes the new context information in sublists which will be sent along with the alert messages, so that certain users will be able to request this new context information without adjustments having to be made to the terminal.

It should also be noted that the central platform 101 in the warning system which is represented in Fig. 1 functions as a gateway. The context information management system 115 stores context information originating from various sources and edits the context information so that it is delivered in optimal form to the terminal on which the context information will be consumed. In this way, having to stream context information directly from an information source to a terminal is avoided and the the context information sources will thus be alleviated.

Although an application of the warning system according to the present invention in a hospital is described above, there are a number of other applications in which the warning system according to the invention provides similar advantages: some advantages of such applications are alerting in the event of fire or smoke detection, alerting in the event of an intrusion in dwellings, offices, industrial buildings, etc., anti-theft systems, tracking people with a tendency to wander, etc.

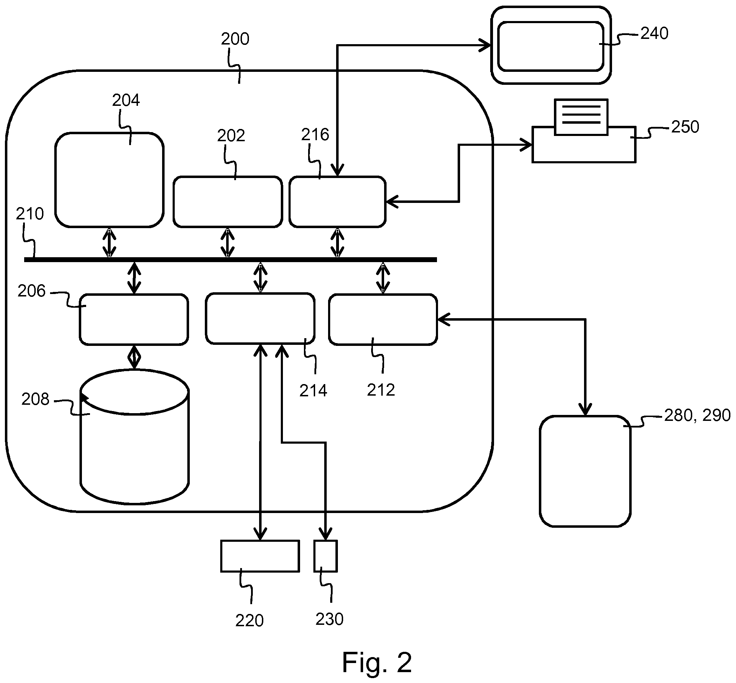

Fig. 2 shows a computer system 200 for carrying out one or more embodiments of the present invention. In particular, central platform 101 from Fig. 1 may be produced on a computer system such as shown in Fig. 2. Computer system 200 may generally take the form of a computer or server suitable for general purposes and comprise a bus 210, a processor 202, a local memory 204, one or more optional input interfaces 214, one or more output interfaces 216, a communication interface 212, a storage element interface 206 and one or more storage elements 208. Bus 210 may comprise one or more conductors, which enable communication between the components of the computer system 200. Processor 202 may comprise any type of conventional processor or microprocessor which interprets and executes program instructions. Local memory 204 may comprise a random access memory (RAM) or another type of dynamic storage device which stores information and instructions for execution by processor 202, and/or a read-only memory (ROM) or another type of static storage device which stores static information and instructions for use by processor 202. Input interface 214 may comprise one or more conventional mechanisms which allow an operator to input information into the computer device 200, such as a keyboard 220, a mouse 230, a stylus, voice recognition and/or biometric mechanisms, etc. Output interface 216 may comprise one or more conventional mechanisms which provide information to the operator, such as a display 240, a printer 250, a speaker, etc. Communication interface 212 may comprise a transceiver-type mechanism, such as for example one or more ethernet interfaces, which allow the computer system 200 to communicate with other devices and/or systems, for example mechanisms for communicating with source and destination systems 141-143 or 102 from Fig. 1. The communication interface 212 of computer system 200 may be connected to such another computer system by means of a local area network (LAN) or a wide area network (WAN), such as for example the internet. Storage element interface 206 may comprise a storage interface, such as for example a Serial Advanced Technology Attachment (SATA) interface or a Small Computer System Interface (SCSI), for connecting bus 210 with one or more storage elements 208, such as one or more local disks, for example SATA disk drives, and for reading and writing data to and/or from these storage elements 208. Although the storage elements 208 above are described as a local disk, in general any other suitable medium that can be read by computer, such as a removable magnetic disk, optical storage media, such as a CD or DVD, CD-ROM, solid-state drives, or flash memory cards, may be used. The system 200 described above may also operate as a virtual machine on top of the physical hardware.

The steps described in the above embodiment(s) may be implemented as program instructions, stored in the local memory 204 of the computer system 200, for execution by the processor 202 thereof. Alternatively, the instructions may be stored in the storage element 208 or be accessible from another computer system via the communication interface 212.

The system 200 may be connected to a network via its communication interface 212. In this way, the system 200 is able to access both the source storage system and the destination storage system 280, 290 for carrying out the steps according to the various embodiments. The steps according to the above embodiments may also be carried out as instructions on one of the servers, in which case these servers have one and the same architecture as the system 200 from Fig. 2. The servers may be managed by a party using the warning system or be managed by a third party, for example a provider and manager of cloud services.

Although the present invention has been illustrated by means of specific embodiments, it will be clear to a person skilled in the art that the invention is not limited to the details of the above illustrative embodiments, and that the present invention may be carried out with various changes and modifications without thereby departing from the area of application of the invention. Therefore, the present embodiments have to be seen in all areas as being illustrative and non-restrictive, and the area of application of the invention is described by the attached claims and not by the above description, and therefore any changes which fall within the meaning and scope of the claims are therefore incorporated herein. In other words, it is assumed that this covers all changes, variations or the like which fall within the area of application of the underlying basic principles and the essential attributes of which are claimed in this patent application. In addition, the reader of this patent application will understand that the terms "comprising" or "comprise" do not exclude other elements or steps, that the term "a(n)/one" does not exclude the plural and that a single element, such as a computer system, a processor or another integrated unit, can perform the functions of different auxiliary means which are mentioned in the claims. Any references in the claims cannot be interpreted as a limitation of the respective claims. The terms "first", "second", "third", "a", "b", "c" and the like, when used in the description or in the claims, are used to distinguish between similar elements or steps and do not necessarily indicate a sequential or chronological order. In the same way, the terms "top side", "bottom side", "above", "below" and the like are used for the sake of the description and do not necessarily refer to relative positions. It should be understood that these terms are interchangeable under the appropriate circumstances and that embodiments of the invention can function according to the present invention in different sequences or orientations than those described or illustrated above.

## Claims

1. Warning system configured for issuing a message relating to an alarming situation, said warning system comprising:
- an alarm distribution module (114) configured to generate an alert message (131) relating to said alarming situation and to send said alert message to one or more terminals (102); and
- a context information management system (115) configured to manage context information related to said alarming situation;
**CHARACTERIZED IN THAT**
- said alarm distribution module (114) is adapted to generate a sublist of requestable context items related to said alarming situation and to include said sublist of requestable context items in said alert message (131), said sublist comprising a reference, indicator or short description of said requestable context items but not comprising said requestable context items themselves, said alarm distribution module (114) being adapted to generate said sublist on the basis of one or more of the following:
- terminal specifications of the terminal (102) to which said alert message (131) is sent;
- user profile specifications of a user of the terminal (102) to which said alert message (131) is sent; and
- legal restrictions; and
- said context information management system (115) is adapted to deliver a context item (133) from among said context information related to said alarming situation only after receiving an explicit request (132) from a terminal (102) from among said terminals.

2. Warning system according to claim 1, further comprising:
- a receiver module (121) in said terminal (102), wherein said receiver module (121) is adapted to receive said alert message (131), to extract the sublist (134) of context items related to said alarming situation from said alert message (131), and to provide the user of said terminal (102) with the possibility of selecting one or more context items from said sublist (134).

3. Warning system according to claim 2, further comprising:
- a request module (122) in said terminal (102), wherein said request module (122) is adapted to generate a request message (132) for one or more context items selected by said user, and to send said request message (132) to said context information management system (115).

4. Warning system according to claim 3, wherein said request module (122) is adapted to also include, in said request message (132), one or more of the following:
- one or more parameters (135) extracted from said alert message (131);
- terminal specifications (136) of the terminal (102) to which said selected context item is to be delivered;
- user preferences (137) indicating an alternative terminal to which said selected context item is to be delivered.

5. Warning system according to one of the preceding claims, wherein different channels are used to send said alert message (131) and to send said selected context item (133).

6. Warning system according to one of the preceding claims, wherein said alarm distribution module (114) sequentially sends said alert message (131):
- to said terminal (102) via alternative channels;
- to alternative terminals associated with one and the same user;
- to alternative terminals in the vicinity of said user; and/or
- to alternative terminals associated with another user who is registered as a backup for said user,
until confirmation of receipt of said alert message (131) by said user is received.

7. Warning system according to one of the preceding claims, wherein said context information management system (115) is adapted to link new sources (141, 142, 143) of context information to said context information management system (115) via plug-in.

8. Warning system according to one of the preceding claims, wherein said context information management system (115) is adapted to receive context information once from a context information source (141, 142, 143), to save, to duplicate and to edit said context information to make variants of said context information which are delivered to one or more terminals in response to explicit requests therefrom.

9. Method, implemented on a computer, for warning about an alarming situation by issuing a message, said method comprising:
- generating and dispatching an alert message (131) relating to said alarming situation from an alarm distribution module (114) to one or more terminals (102); and
- managing context information related to said alarming situation in a context information management system (115);
**CHARACTERIZED IN THAT** said method further comprises:
- generating a sublist of requestable context items related to said alarming situation and including said sublist of requestable context items in said alert message (131), said sublist comprising a reference, indicator or short description of said requestable context items but not comprising said requestable context items themselves, said sublist being generated on the basis of one or more of the following:
- terminal specifications of the terminal (102) to which said alert message (131) is sent;
- user profile specifications of a user of the terminal (102) to which said alert message (131) is sent; and
- legal restrictions; and
- sending a context item (133) from among said context information related to said alarming situation by said context management system (115) only after receiving an explicit request (132) from a terminal (102) from among said terminals.

10. Computer program product comprising instructions that can be run on a computer to carry out the method according to claim 9 when said program is run on a computer.

11. A storage medium that can be read by a computer, said storage medium comprising the computer program product according to claim 10.

## Patentansprüche

1. Warnsystem, das konfiguriert ist, um eine sich auf eine Alarmsituation beziehende Nachricht auszugeben, wobei das Warnsystem umfasst:
- ein Alarmverteilungsmodul (114), das konfiguriert ist, um eine Warnnachricht (131) zu generieren, die sich auf die Alarmsituation bezieht, und die Alarmnachricht an ein oder mehrere Endgeräte (102) zu senden; und
- ein Kontextinformationsverwaltungssystem (115), das konfiguriert ist, um auf die Alarmsituation bezogene Kontextinformationen zu verwalten;
**DADURCH GEKENNZEICHNET, DASS**
- das Alarmverteilungsmodul (114) angepasst ist, um eine Unterliste anfragbarer Kontextartikel zu generieren, die sich auf die Alarmsituation beziehen, und um die Unterliste anfragbarer Kontextartikel in die Alarmnachricht (131) einzubinden, wobei die Unterliste eine Referenz, einen Indikator oder eine Kurzbeschreibung der anfragbaren Kontextartikel umfasst, aber nicht dieselben anfragbaren Kontextartikel selbst umfasst, wobei das Alarmverteilungsmodul (114) angepasst ist, um die Unterliste auf der Basis eines oder mehrerer der Folgenden zu generieren:
- Endgerätspezifikationen des Endgeräts (102), an das die Alarmnachricht (131) gesendet wird;
- Nutzerprofilspezifikationen eines Nutzers des Endgeräts (102), an den die Alarmnachricht (131) gesendet wird; und
- rechtliche Einschränkungen; und
- das Kontextinformationsverwaltungssystem (115) angepasst ist, um einen Kontextartikel (133) unter den auf die Alarmsituation bezogenen Kontextinformationen erst nach Empfangen einer ausdrücklichen Anfrage (132) von einem Endgerät (102) unter den Endgeräten zu liefern.

2. Warnsystem nach Anspruch 1, ferner umfassend:
- ein Empfängermodul (121) im Endgerät (102), wobei das Empfängermodul (121) angepasst ist, um die Alarmnachricht (131) zu empfangen, um die Subliste (134) von Kontextartikeln, die sich auf die Alarmsituation beziehen, aus der Alarmnachricht (131) zu extrahieren, und dem Nutzer des Endgeräts (102) die Möglichkeit bereitzustellen, einen oder mehrere Kontextartikel aus der Subliste (134) auszuwählen.

3. Warnsystem nach Anspruch 2, ferner umfassend:
- ein Anfragemodul (122) im Endgerät (102), wobei das Anfragemodul (122) angepasst ist, um eine Anfragenachricht (132) für einen oder mehrere vom Nutzer ausgewählte Kontextartikel zu generieren und die Anfragenachricht (132) an das Kontextinformationsverwaltungssystem (115) zu senden.

4. Warnsystem nach Anspruch 3, wobei das Anfragemodul (122) angepasst ist, um in der Anfragenachricht (132) auch eines oder mehrere der Folgenden zu enthalten:
- einen oder mehrere aus der Alarmnachricht (131) extrahierte Parameter (135);
- Endgerätespezifikationen (136) des Endgeräts (102), an den der ausgewählte Kontextartikel zu liefern ist;
- Nutzerpräferenzen (137), die ein alternatives Endgerät anzeigen, an das der ausgewählte Kontextartikel zu liefern ist.

5. Warnsystem nach einem der vorangehenden Ansprüche, wobei unterschiedliche Kanäle genutzt werden, um Alarmnachrichten (131) zu senden und den ausgewählten Inhaltsartikel (133) zu senden.

6. Warnsystem nach einem der vorangehenden Ansprüche, wobei das Alarmverteilungsmodul (114) aufeinanderfolgend die Alarmnachricht (131) sendet:
- an das Endgerät (102) über alternative Kanäle;
- an alternative Endgeräte, die einem und demselben Nutzer zugeordnet sind;
- an alternative Endgeräte in der Nähe des Nutzers; und/oder
- an alternative Endgeräte, die einem anderen Nutzer zugeordnet sind, der als ein Backup für den Nutzer registriert ist, bis eine Empfangsbestätigung der Alarmnachricht (131) vom Nutzer empfangen wird.

7. Warnsystem nach einem der vorangehenden Ansprüche, wobei das Kontextinformationsverwaltungssystem (115) angepasst ist, um neue Quellen (141, 142, 143) von Kontextinformationen an das Kontextinformationsverwaltungssystem (115) über ein Plug-in zu verknüpfen.

8. Warnsystem nach einem der vorangehenden Ansprüche, wobei das Kontextinformationsverwaltungssystem (115) angepasst ist, um Kontextinformationen einmal von einer Kontextinformationsquelle (141, 142, 143) zu empfangen, die Kontextinformationen zu speichern, zu duplizieren und zu bearbeiten, um Varianten der Kontextinformationen zu erstellen, die in Reaktion auf ausdrückliche Anfragen davon an ein oder mehr Endgeräte zu liefern sind.

9. Verfahren, das auf einem Computer implementiert ist, zum Warnen über eine Alarmsituation durch Ausgeben einer Nachricht, wobei das Verfahren umfasst:
- ein Generieren und Abschicken einer Alarmnachricht (131), die sich auf die Alarmsituation bezieht, von einem Alarmverteilungsmodul (114) an ein oder mehrere Endgeräte (102); und
- ein Verwalten von Kontextinformationen, die sich auf die Alarmsituation beziehen, in einem Kontextinformationsverwaltungssystem (115);
**DADURCH GEKENNZEICHNET, dass** das Verfahren ferner umfasst:
- ein Generieren einer Unterliste anfragbarer Kontextartikel, die sich auf die Alarmsituation beziehen, und ein Einbinden der Unterliste anfragbarer Kontextartikel in die Alarmnachricht (131), wobei die Unterliste eine Referenz, einen Indikator oder eine Kurzbeschreibung der anfragbaren Kontextartikel umfasst, aber nicht dieselben anfragbaren Kontextartikel selbst umfasst, wobei die Unterliste auf der Basis einer oder mehrerer der Folgenden generiert wird:
- Endgerätspezifikationen des Endgeräts (102), an das die Alarmnachricht (131) gesendet wird;
- Nutzerprofilspezifikationen eines Nutzers des Endgeräts (102), an den die Alarmnachricht (131) gesendet wird; und
- rechtliche Einschränkungen; und
- ein Senden eines Kontextartikels (133) unter den auf die Alarmsituation bezogenen Kontextinformationen durch das Kontextverwaltungssystem (115) erst nach dem Empfangen einer ausdrücklichen Anfrage (132) von einem Endgerät (102) unter den Endgeräten.

10. Computerprogrammprodukt, umfassend Anweisungen, die auf einem Computer durchlaufen werden können, um das Verfahren nach Anspruch 9 auszuführen, wenn das Programm auf einem Computer durchlaufen wird.

11. Speichermedium, das von einem Computer gelesen werden kann, wobei das Speichermedium das Computerprogrammprodukt nach Anspruch 10 umfasst.

## Revendications

1. Système d'avertissement conçu pour émettre un message relatif à une situation alarmante, ledit système d'avertissement comprenant :
- un module de distribution d'alarme (114) conçu pour générer un message d'alerte (131) relatif à ladite situation alarmante et pour envoyer ledit message d'alerte à un ou plusieurs terminaux (102) ; et
- un système de gestion d'informations de contexte (115) configuré pour gérer des informations de contexte liées à ladite situation alarmante ;
**CARACTERISE EN CE QUE**
- ledit module de distribution d'alarme (114) est adapté pour générer une sous-liste d'éléments de contexte pouvant être demandés liés à ladite situation alarmante et pour comprendre ladite sous-liste d'éléments de contexte pouvant être demandés dans ledit message d'alerte (131), ladite sous-liste comprenant une référence, un indicateur ou une brève description desdits éléments de contexte pouvant être demandés mais ne comprenant pas lesdits éléments de contexte pouvant être demandés eux-mêmes, ledit module de distribution d'alarme (114) étant adapté pour générer ladite sous-liste sur la base de l'un ou plusieurs des suivants :
- les spécifications de terminal du terminal (102) auquel ledit message d'alerte (131) est envoyé ;
- des spécifications de profil d'utilisateur d'un utilisateur du terminal (102) auquel ledit message d'alerte (131) est envoyé ; et
- des restrictions légales ; et
- ledit système de gestion d'informations de contexte (115) est adapté pour délivrer un élément de contexte (133) parmi lesdites informations de contexte liées à ladite situation alarmante uniquement après avoir reçu une demande explicite (132) en provenance d'un terminal (102) parmi lesdits terminaux.

2. Système d'avertissement selon la revendication 1, comprenant en outre :
- un module récepteur (121) dans ledit terminal (102), ledit module récepteur (121) étant adapté pour recevoir ledit message d'alerte (131), pour extraire la sous-liste (134) des éléments de contexte liés à ladite situation alarmante dudit message d'alerte (131), et pour offrir à l'utilisateur dudit terminal (102) la possibilité de choisir un ou plusieurs éléments de contexte dans ladite sous-liste (134).

3. Système d'avertissement selon la revendication 2, comprenant en outre :
- un module de demande (122) dans ledit terminal (102), ledit module de demande (122) étant adapté pour générer un message de demande (132) pour un ou plusieurs éléments de contexte choisis par ledit utilisateur, et pour envoyer ledit message de demande (132) audit système de gestion d'informations de contexte (115).

4. Système d'avertissement selon la revendication 3, ledit module de demande (122) étant adapté pour comprendre également, dans ledit message de demande (132), l'un ou plusieurs des suivants :
- un ou plusieurs paramètres (135) extraits dudit message d'alerte (131) ;
- les spécifications de terminal (136) du terminal (102) auquel ledit élément de contexte choisi doit être délivré ;
- les préférences d'utilisateur (137) indiquant un terminal alternatif auquel ledit élément de contexte choisi doit être livré.

5. Système d'avertissement selon l'une des revendications précédentes, différents canaux étant utilisés pour envoyer ledit message d'alerte (131) et pour envoyer ledit élément de contexte choisi (133).

6. Système d'avertissement selon l'une des revendications précédentes, ledit module de distribution d'alarme (114) envoyant séquentiellement ledit message d'alerte (131) :
- vers ledit terminal (102) par l'intermédiaire des canaux alternatifs ;
- vers des terminaux alternatifs associés à un et même utilisateur ;
- à des terminaux alternatifs à proximité dudit utilisateur ; et/ou
- à des terminaux alternatifs associés à un autre utilisateur qui est enregistré en tant que sauvegarde pour ledit utilisateur, jusqu'à la confirmation de la réception dudit message d'alerte (131) par ledit utilisateur.

7. Système d'avertissement selon l'une des revendications précédentes, ledit système de gestion d'informations de contexte (115) étant adapté pour lier de nouvelles sources (141, 142, 143) d'informations de contexte audit système de gestion d'informations de contexte (115) par l'intermédiaire d'un module d'extension.

8. Système d'avertissement selon l'une des revendications précédentes, ledit système de gestion d'informations de contexte (115) étant adapté pour recevoir une fois des informations de contexte en provenance d'une source d'informations de contexte (141, 142, 143), pour enregistrer, dupliquer et éditer lesdites informations de contexte pour réaliser des variantes desdites informations de contexte qui sont délivrées à un ou plusieurs terminaux en réponse à des demandes explicites en provenance de ceux-ci.

9. Procédé, mis en oeuvre sur un ordinateur, permettant d'avertir d'une situation alarmante en émettant un message, ledit procédé comprenant :
- la génération et l'envoi d'un message d'alerte (131) relatif à ladite situation alarmante à partir d'un module de distribution d'alarme (114) vers un ou plusieurs terminaux (102) ; et
- la gestion des informations de contexte liées à ladite situation alarmante dans un système de gestion d'informations de contexte (115) ;
**CARACTERISE EN CE QUE** ledit procédé comprend en outre :
- la génération d'une sous-liste d'éléments de contexte pouvant être demandés liés à ladite situation alarmante et comprenant ladite sous-liste des éléments de contexte pouvant être demandés dans ledit message d'alerte (131), ladite sous-liste comprenant une référence, un indicateur ou une brève description desdits éléments de contexte pouvant être demandés mais ne comprenant pas lesdits éléments de contexte, pouvant être demandés, eux-mêmes, ladite sous-liste étant générée sur la base de l'un ou plusieurs des suivants :
- les spécifications de terminal du terminal (102) auquel ledit message d'alerte (131) est envoyé ;
- des spécifications de profil d'utilisateur d'un utilisateur du terminal (102) auquel ledit message d'alerte (131) est envoyé ; et
- des restrictions légales ; et
- l'envoi d'un élément de contexte (133) parmi lesdites informations de contexte liées à ladite situation alarmante par ledit système de gestion de contexte (115) uniquement après avoir reçu une demande explicite (132) en provenance d'un terminal (102) parmi lesdits terminaux.

10. Produit de programme informatique comprenant des instructions qui peuvent être exécutées sur un ordinateur pour effectuer le procédé selon la revendication 9 lorsque ledit programme est exécuté sur un ordinateur.

11. Support de stockage qui peut être lu par un ordinateur, ledit support de stockage comprenant le produit de programme informatique selon la revendication 10.
